# EUROPEAN PATENT APPLICATION

(11) **EP 1 938 741 A1**
(43) Date of publication of application: **02.07.2008**
(21) Application number: 06782982.0
(22) Date of filing: 24.08.2006
(51) Int. Cl.: A61B 1/04, A61B 1/00, G02B 23/24, G02B 23/26, H04N 7/18

(54) **ORGANISM IMAGING DEVICE AND ORGANISM OBSERVING SYSTEM**

(30) Priority: 21.10.2005 JP 2005307620
(71) Applicant: OLYMPUS MEDICAL SYSTEMS CORP., Shibuya-ku Tokyo 151-0072 (JP)
(72) Inventor: GONO, Kazuhiro c/o Olympus Medical Systems Corp., Shibuya-ku, Tokyo 151-0072 (JP)
(74) Representative: von Hellfeld, Axel
(86) International application number: PCT/JP2006/316578
(87) International publication number: WO 2007/046188

(57) **Abstract**

A living body imaging system which can reduce costs for narrow band imaging more than before is provided. A living body image pickup apparatus according to the present invention includes: a plurality of image pickup units which pick up images of body tissue illuminated by a white light and output the images of the body tissue as image pickup signals; a first spectroscopic unit which passes light in a first wavelength band, spectrally analyzes an image of the body tissue picked up by a first image pickup unit out of the plurality of image pickup units and thereby allows the image of the body tissue to be displayed on a display unit as a first image; and a second spectroscopic unit which passes light in a second wavelength band different from the first wavelength band, spectrally analyzes an image of the body tissue picked up by a second image pickup unit out of the plurality of image pickup units and thereby allows the image of the body tissue to be displayed on the display unit as a second image in which predetermined part of the body tissue is enhanced compared to the first image.

## Description

### Technical Field

The present invention relates to a living body image pickup apparatus and living body imaging system, and more particularly, to a living body image pickup apparatus and living body imaging system capable of picking up images with predetermined part enhanced.

### Background Art

Endoscope apparatuses equipped with an endoscope and a light source and so on have been widely used in the medical field. In particular, the endoscope apparatus in the medical field are mainly used by surgeons and the like to observe inside of a living body which is an object to be examined.

Known observation methods using an endoscope apparatus in the medical field include normal imaging which involves illuminating an imaging subject in a living body with white light and picking up images of the living body similar to images obtained by naked-eye observations, and narrow band imaging (NBI) which involves making observations by illuminating the imaging subject with narrow-band light narrower than the illuminating light used for the normal imaging and thereby picking up images in which blood vessels and the like in a surface layer of mucous membrane on the living body are more enhanced than in the normal imaging.

An endoscope system disclosed in Japanese Patent Laid-Open Application No. 2002-095635 includes a light source equipped with filters with discrete spectral characteristics to output narrow-band illuminating light and an endoscope used to pick up images of an imaging subject illuminated by the illuminating light. The above configuration allows the endoscope system disclosed in Japanese Patent Laid-Open Application No. 2002-095635 to perform narrow band imaging of the imaging subject.

However, to perform narrow band imaging, the endoscope system disclosed in Japanese Patent Laid-Open Application No. 2002-095635 requires the light source which, being equipped with filters with discrete spectral characteristics, supports narrow band imaging. This increases costs of manufacturing the light source equipped with the filters, resulting in increased costs for narrow band imaging of imaging subjects.

The present invention has been made in view of the above circumstances and has an object to provide a living body image pickup apparatus and living body imaging system which can reduce costs for narrow band imaging more than before.

### Disclosure of Invention

### Means for Solving the Problem

A first living body image pickup apparatus according to the present invention comprises: a plurality of image pickup means which pick up images of body tissue illuminated by a white light and output the images of the body tissue as image pickup signals; first spectroscopic means which, having transmission characteristics of passing light in a first wavelength band, spectrally analyzes an image of the body tissue picked up by first image pickup means out of the plurality of image pickup means and thereby allows the image of the body tissue to be displayed on display means as a first image; and second spectroscopic means which, having transmission characteristics of passing light in a second wavelength band different from the first wavelength band, spectrally analyzes an image of the body tissue picked up by second image pickup means out of the plurality of image pickup means and thereby allows the image of the body tissue to be displayed on the display means as a second image in which predetermined part of the body tissue is enhanced compared to the first image.

A second living body image pickup apparatus according to the present invention is the first living body image pickup apparatus, further comprising third spectroscopic means which, having transmission characteristics of passing light in a third wavelength band different from the first wavelength band and the second wavelength band, spectrally analyzes an image of the body tissue picked up by third image pickup means out of the plurality of image pickup means and thereby allows the image of the body tissue to be displayed on the display means as a third image in which predetermined part is enhanced compared to the second image.

A third living body image pickup apparatus according to the present invention is the first or second living body image pickup apparatus, wherein the predetermined part is blood vessels.

A fourth living body image pickup apparatus according to the present invention is any one of the first to third living body image pickup apparatus, wherein the light in the first wavelength band includes a red band, a first green band, and a first blue band.

A fifth living body image pickup apparatus according to the present invention is the fourth living body image pickup apparatus, wherein the light in the second wavelength band includes a second green band narrower than the first green band and a second blue band narrower than the first blue band.

A sixth living body image pickup apparatus according to the present invention is the fourth or fifth living body image pickup apparatus, wherein the light in the third wavelength band has a plurality of peak wavelengths in the first blue band.

A first living body imaging system according to the present invention comprises: a light source which emits a white light as an illuminating light to illuminate an imaging subject in a living body; a living body image pickup apparatus which picks up images of the imaging subject and outputs the images of the imaging subject as an image pickup signal; and a controller which controls the light source and the living body image pickup apparatus, wherein the living body image pickup apparatus comprises a plurality of image pickup means which pick up images of the imaging subject and output the images of the imaging subject as image pickup signals, first spectroscopic means which, having transmission characteristics of passing light in a first wavelength band, spectrally analyzes an image of the imaging subject picked up by first image pickup means out of the plurality of image pickup means and thereby allows the image of the imaging subject to be displayed on display means as a first image, and second spectroscopic means which, having transmission characteristics of passing light in a second wavelength band different from the first wavelength band, spectrally analyzes an image of the imaging subject picked up by second image pickup means out of the plurality of image pickup means and thereby allows the image of the imaging subject to be displayed on the display means as a second image in which predetermined body tissue in the imaging subject is enhanced compared to the first image, and the controller comprises user control means which outputs an imaging mode switching signal to switch between a first imaging mode for imaging of the first image and a second imaging mode for imaging of the second image, and control means which outputs a control signal if it is detected based on the imaging mode switching signal that imaging in one of the imaging modes is specified from the user control means, the control signal being intended to adjust exposure time of each of the plurality of image pickup means, control light quantity of the white light emitted by the light source means, and change display state of the first image and the second image on the display means, according to the specified imaging mode.

A second living body imaging system according to the present invention is the first living body imaging system, wherein the predetermined part is blood vessels.

A third living body imaging system according to the present invention is the first or second living body imaging system, wherein the light in the first wavelength band includes a red band, a first green band, and a first blue band.

A fourth living body imaging system according to the present invention is the third living body imaging system, wherein the light in the second wavelength band includes a second green band narrower than the first green band and a second blue band narrower than the first blue band.

### Brief Description of the Drawings

Fig. 1 is a diagram showing an exemplary configuration of a principal part of a living body imaging system in which a living body image pickup apparatus according to the present embodiment is used;
Fig. 2 is a diagram showing a relationship between wavelength and transmittance of a spectral filter in the living body image pickup apparatus in Fig. 1;
Fig. 3 is a diagram showing a relationship between wavelength and transmittance of a spectral filter in the living body image pickup apparatus in Fig. 1, where the spectral filter passes light in a wavelength band different from the wavelength band in Fig. 2;
Fig. 4 is a diagram showing an example of images displayed in normal imaging mode and narrow band imaging mode on a monitor of the living body imaging system in Fig. 1;
Fig. 5 is a diagram showing another exemplary configuration of a principal part of the living body imaging system in which the living body image pickup apparatus according to the present embodiment is used;
Fig. 6 is a diagram showing a relationship between wavelength and transmittance of a spectral filter in the living body image pickup apparatus in Fig. 5, where the spectral filter is different from the spectral filters in Figs. 2 and 3; and
Fig. 7 is a diagram showing an example of images displayed in normal imaging mode, first narrow band imaging mode, and second narrow band imaging mode on a monitor of the living body imaging system in Fig. 5.

### Best Mode for Carrying Out the Invention

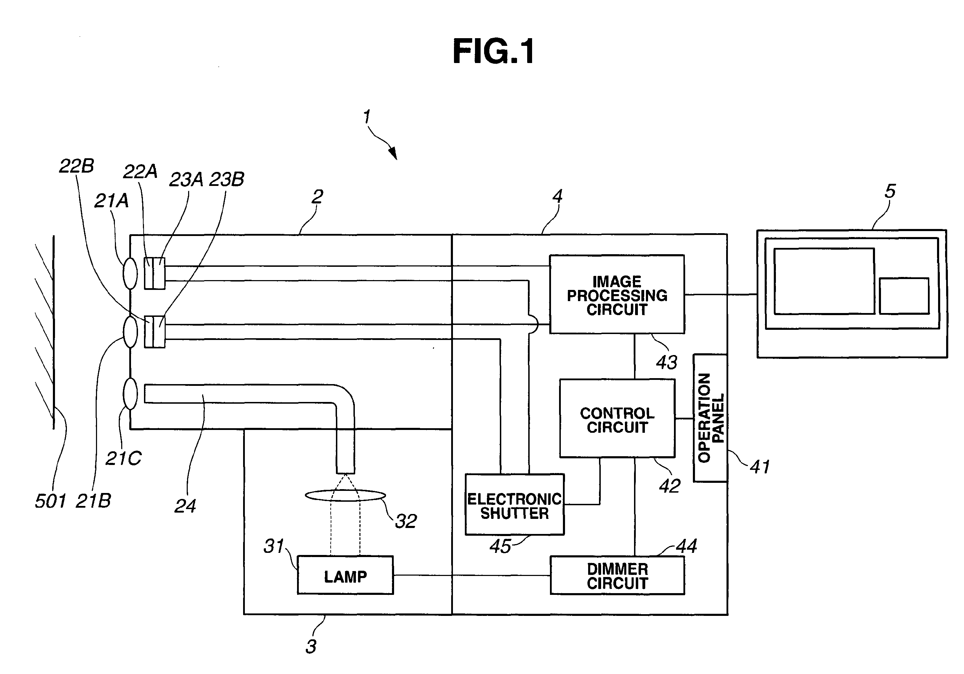

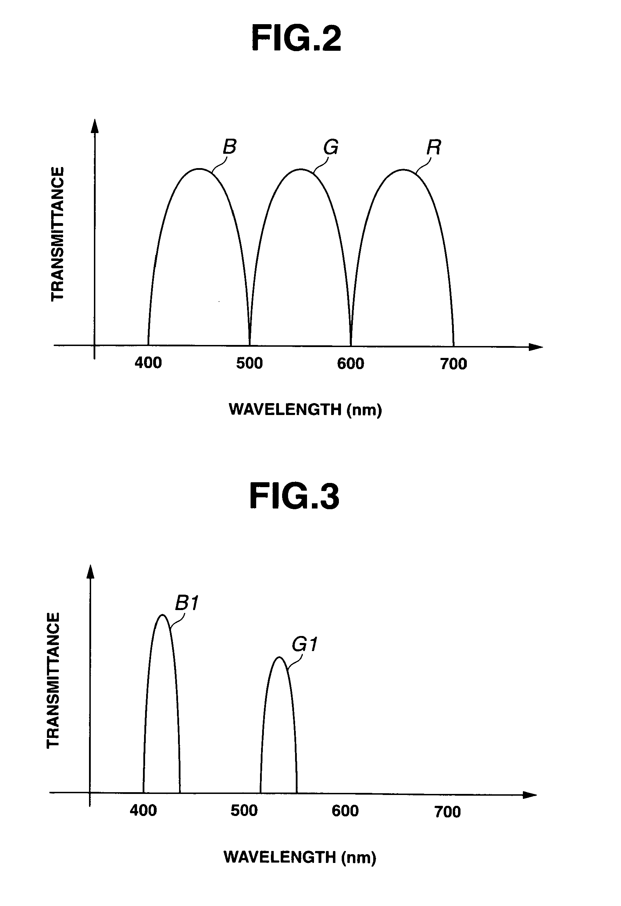

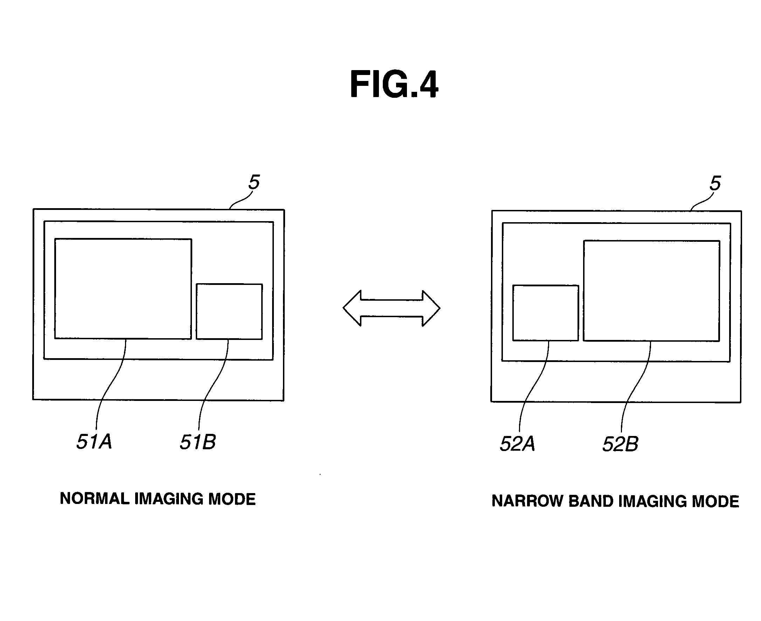

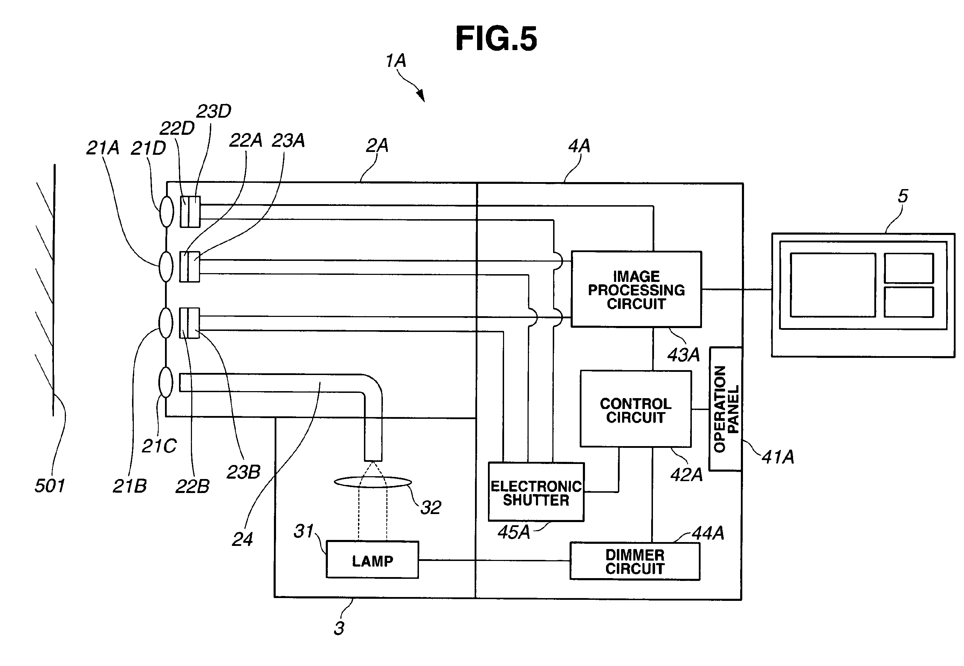

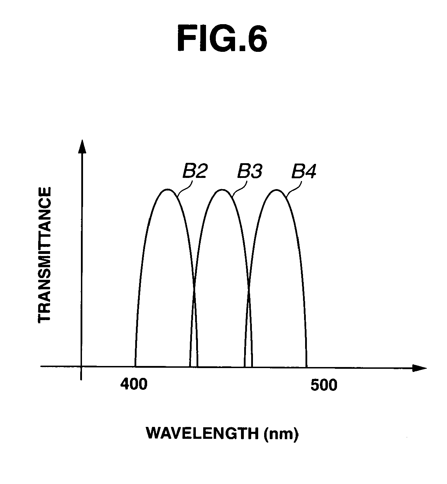

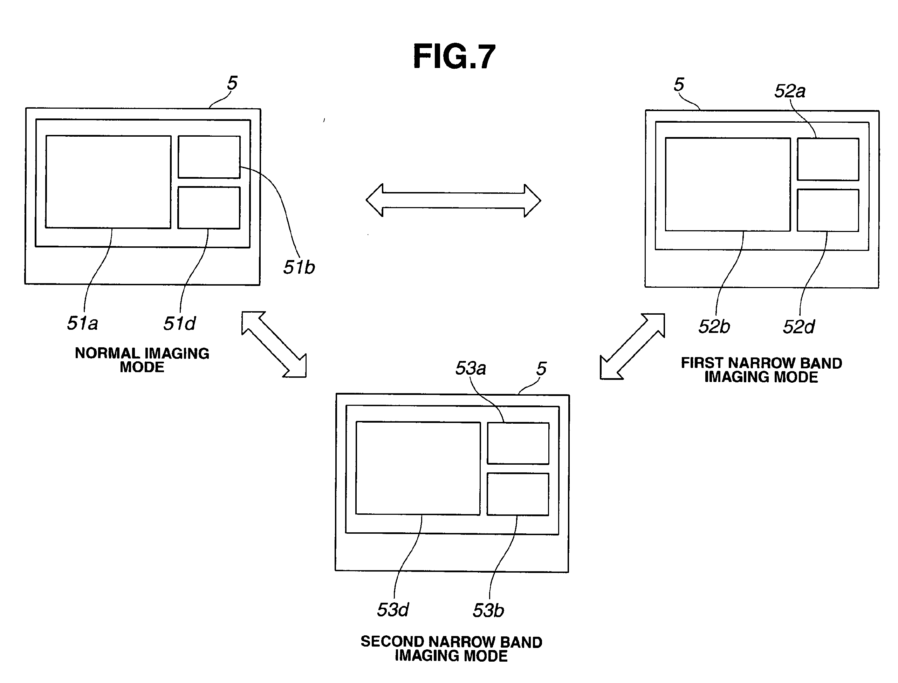

Figs. 1 to 7 relate to an embodiment of the present invention. Fig. 1 is a diagram showing an exemplary configuration of a principal part of a living body imaging system in which a living body image pickup apparatus according to the present embodiment is used. Fig. 2 is a diagram showing a relationship between wavelength and transmittance of a spectral filter in the living body image pickup apparatus in Fig. 1. Fig. 3 is a diagram showing a relationship between wavelength and transmittance of a spectral filter in the living body image pickup apparatus in Fig. 1, where the spectral filter passes light in a wavelength band different from the wavelength band in Fig. 2. Fig. 4 is a diagram showing an example of images displayed in normal imaging mode and narrow band imaging mode on a monitor of the living body imaging system in Fig. 1. Fig. 5 is a diagram showing another exemplary configuration of a principal part of the living body imaging system in which the living body image pickup apparatus according to the present embodiment is used. Fig. 6 is a diagram showing a relationship between wavelength and transmittance of a spectral filter in the living body image pickup apparatus in Fig. 5, where the spectral filter is different from the spectral filters in Figs. 2 and 3. Fig. 7 is a diagram showing an example of images displayed in normal imaging mode, first narrow band imaging mode, and second narrow band imaging mode on a monitor of the living body imaging system in Fig. 5.

As shown in Fig. 1, a principal part of a living body imaging system 1 includes a living body image pickup apparatus 2 which picks up images of an imaging subject 501 such as body tissues in a living body which is an object to be examined and outputs the images as an image pickup signal, a light source 3 which supplies an illuminating light to the imaging subject 501 whose images are picked up by the living body image pickup apparatus 2, a controller 4 which generates and outputs a video signal based on the image pickup signal outputted by the living body image pickup apparatus 2 and controls the light source 3, and a monitor 5 serving as display means which displays the images of the imaging subject 501 picked up by the living body image pickup apparatus 2, based on the video signal outputted by the controller 4.

The living body image pickup apparatus 2 configured as an endoscope or the like includes an objective optical system 21A which forms an image of the imaging subject 501, a spectral filter 22A which passes light in a first wavelength band and spectrally analyzes the image of the imaging subject 501 focused by the objective optical system 21A, and an image pickup device 23A which picks up the image of the imaging subject 501 spectrally analyzed by the spectral filter 22A and outputs the resulting image of the imaging subject 501 as an image pickup signal, the image pickup device 23A including a CCD (charge-coupled device) or the like and serving as image pickup means. The objective optical system 21A, the spectral filter 22A, and image pickup device 23A make up normal imaging means.

Also, the living body image pickup apparatus 2 includes an objective optical system 21B which forms an image of the imaging subject 501, a spectral filter 22B which passes light in a second wavelength band and spectrally analyzes the image of the imaging subject 501 focused by the objective optical system 21B, and an image pickup device 23B which picks up the image of the imaging subject 501 spectrally analyzed by the spectral filter 22B and outputs the resulting image of the imaging subject 501 as an image pickup signal, the image pickup device 23B including a CCD (charge-coupled device) or the like and serving as image pickup means. The objective optical system 21B, the spectral filter 22B, and image pickup device 23B make up first narrow band imaging means.

Furthermore, the living body image pickup apparatus 2 includes an illumination objective system 21C which emits an illumination light and a light guide 24 which leads an illuminating light emitted by the light source 3 to the illumination objective system 21C.

The spectral filter 22A which is made up of a mosaic filter or band-limiting filter and serving as spectroscopic means, has transmission characteristics of passing light in the first wavelength band including, for example, R, G, and B wavelength bands shown in Fig. 2. Specifically, the R wavelength band ranges between 600 nm and 700 nm, G wavelength band ranges between 500 nm and 600 nm, and B wavelength band ranges between 400 nm and 500 nm. The first wavelength band may be designed to approximately correspond to primary color filters as described above or to complementary color filters.

The spectral filter 22B which is made up of a mosaic filter or band-limiting filter and serving as spectroscopic means, has transmission characteristics of passing light in the second wavelength band including, for example, a G1 wavelength band narrower than the G wavelength band and a B 1 wavelength band narrower than the B wavelength band shown in Fig. 3. Specifically, the G1 wavelength band ranges between 530 nm and 560 nm and B1 wavelength band ranges between 400 nm and 430.

The light source 3 is made up of a xenon lamp or the like which emits a white light as an illuminating light. The light source 3 includes a lamp 31 serving as light source means and capable of changing quantity of emitted light under the control of the controller 4 and a condenser optical system 32 which gathers the white light emitted by the lamp 31 and thereby supplies the illuminating light to the light guide 24 to illuminate the imaging subject 501.

The controller 4 is installed on an exterior surface of the controller 4. The controller 4 includes an operation panel 41 which outputs command signals in response to surgeon's actions, a control circuit 42 which performs various types of control based on the command signals outputted from the operation panel 41, an image processing circuit 43 which processes the image pickup signals outputted from the image pickup devices 23A and 23B under the control of the control circuit 42, a dimmer circuit 44 which controls the quantity of light emitted by the lamp 31 of the light source 3 under the control of the control circuit 42, and an electronic shutter 45 which adjusts exposure times of the image pickup devices 23A and 23B.

The operation panel 41 includes user control means such as an imaging mode changeover switch which outputs an imaging mode changeover command signal in order to switch between normal imaging mode which provides images of a desired imaging subject in a living body in a manner similar to naked-eye observations and narrow band imaging mode which provides images with blood vessels and microstructures in a surface layer of mucous membrane on the imaging subject in the living body being enhanced.

If, for example, a command for normal imaging is detected based on the command signal outputted from the operation panel 41, the control circuit 42 serving as control means outputs a first control signal containing control details for the normal imaging mode to the image processing circuit 43, dimmer circuit 44, and electronic shutter 45.

Also, if, for example, a command for narrow band imaging mode is detected based on the command signal outputted from the operation panel 41, the control circuit 42 outputs a second control signal containing control details for the narrow band imaging mode to the image processing circuit 43, dimmer circuit 44, and electronic shutter 45.

Based on the first control signal outputted from the control circuit 42, the image processing circuit 43 serving as image processing means processes the image pickup signals outputted from the image pickup devices 23A and 23B so that the image of the imaging subject 501 picked up by the image pickup device 23A will be displayed in a larger size on the monitor 5 than the image of the imaging subject 501 picked up by the image pickup device 23B, and outputs the processed image pickup signals to the monitor 5.

Also, based on the second control signal outputted from the control circuit 42, the image processing circuit 43 processes the image pickup signals outputted from the image pickup devices 23A and 23B so that the image of the imaging subject 501 picked up by the image pickup device 23B will be displayed in larger sizes on the monitor 5 than the image of the imaging subject 501 picked up by the image pickup device 23A, and outputs the processed image pickup signals as video signals to the monitor 5.

Based on the first control signal outputted from the control circuit 42, the dimmer circuit 44 serving as dimming means sets the quantity of light emitted by the lamp 31 to a first emitted-light quantity so that the image of the imaging subject 501 picked up by the image pickup device 23A will be displayed on the monitor 5 at a brightness suitable for normal imaging.

Also, based on the second control signal outputted from the control circuit 42, the dimmer circuit 44 sets the quantity of light emitted by the lamp 31 to a second emitted-light quantity larger than the first emitted-light quantity so that the image of the imaging subject 501 picked up by the image pickup device 23B will be displayed on the monitor 5 at a brightness suitable for the narrow band imaging.

Based on the first control signal outputted from the control circuit 42, the electronic shutter 45 serving as exposure adjusting means sets the exposure time of the image pickup device 23A to a first exposure time suitable for the normal imaging and sets the exposure time of the image pickup device 23B to a second exposure time longer than the first exposure time.

Also, based on the second control signal outputted from the control circuit 42, the electronic shutter 45 sets the exposure time of the image pickup device 23B to a third exposure time suitable for the narrow band imaging and sets the exposure time of the image pickup device 23A to a fourth exposure time shorter than the third exposure time.

Next, operation of the living body imaging system 1 according to the present embodiment will be described.

The surgeon or the like powers on various components of the living body imaging system 1, i.e., the living body image pickup apparatus 2, light source 3, controller 4, and monitor 5 and thereby starts up these components. It is assumed that the living body image pickup apparatus 2, light source 3, and controller 4 are set to enter normal imaging mode upon start-up.

When the controller 4 is set to normal imaging mode, the control circuit 42 detects based on the imaging mode changeover command signal outputted from the operation panel 41 that normal imaging has been specified, and outputs the first control signal to the image processing circuit 43, dimmer circuit 44, and electronic shutter 45. Also, when the controller 4 is set to the normal imaging mode, based on the first control signal outputted from the control circuit 42, the dimmer circuit 44 sets the quantity of light emitted by the lamp 31 to the first emitted-light quantity so that the image of the imaging subject 501 picked up by the image pickup device 23A will be displayed on the monitor 5 at a brightness suitable for the normal imaging. Furthermore, when the controller 4 is set to the normal imaging mode, based on the first control signal outputted from the control circuit 42, the electronic shutter 45 sets the exposure time of the image pickup device 23A to the first exposure time suitable for the normal imaging and sets the exposure time of the image pickup device 23B to the second exposure time longer than the first exposure time.

Subsequently, the surgeon or the like moves the living body image pickup apparatus 2 to place a desired imaging subject in the living body in such a position as to come into view of the objective optical systems 21A and 21B and be illuminated with the illuminating light emitted from the illumination objective system 21C.

Under the conditions described above, images of the imaging subject 501 illuminated with a wide band light emitted by the illumination objective system 21C are respectively focused by the objective optical systems 21A and 21B, spectrally analyzed by the spectral filters 22A and 22B, picked up by the image pickup devices 23A and 23B, and outputted as image pickup signals to the image processing circuit 43 of the controller 4.

Based on the first control signal outputted from the control circuit 42 and the image pickup signals outputted, respectively, from the image pickup devices 23A and 23B, the image processing circuit 43 processes the image pickup signals outputted from the image pickup devices 23A and 23B so that the image of the imaging subject 501 picked up by the image pickup device 23A will be displayed in larger sizes on the monitor 5 than the image of the imaging subject 501 picked up by the image pickup device 23B, and outputs the processed image pickup signals as video signals to the monitor 5.

Based on the video signals outputted from the image processing circuit 43, the monitor 5 displays the image of the imaging subject 501 picked up by the image pickup device 23A as a normal image 51A and the image of the imaging subject 501 picked up by the image pickup device 23B as a narrow band image 51B, for example, as shown in Fig. 4.

As the control, processes, and the like described above are performed by the living body image pickup apparatus 2, light source 3, and controller 4, images are displayed on the monitor 5 in a form suitable for display in normal imaging mode: the normal image 51 A is displayed in an enlarged form at an optimum brightness and the narrow band image 51 B is displayed in a reduced form. Furthermore, as the control, processes, and the like described above are performed by the living body image pickup apparatus 2, light source 3, and controller 4, the normal image 51A is displayed on the monitor 5 in a manner similar to naked-eye observations of a desired imaging subject in the living body.

When the surgeon or the like gives a command, for example, to switch the imaging mode of the living body image pickup apparatus 2, light source 3, and controller 4 from normal imaging mode to narrow band imaging mode by manipulating the imaging mode changeover switch on the operation panel 41, the operation panel 41 outputs an imaging mode changeover command signal to the control circuit 42 based on the command.

Based on the imaging mode changeover command signal outputted from the operation panel 41, the control circuit 42 detects that narrow band imaging has been specified, and outputs the second control signal to the image processing circuit 43, dimmer circuit 44, and electronic shutter 45. Also, when the controller 4 is set to the narrow band imaging mode, based on the second control signal outputted from the control circuit 42, the dimmer circuit 44 sets the quantity of light emitted by the lamp 31 to the second emitted-light quantity so that the image of the imaging subject 501 picked up by the image pickup device 23A will be displayed on the monitor 5 at a brightness suitable for the narrow band imaging. Furthermore, when the controller 4 is set to the narrow band imaging mode, based on the second control signal outputted from the control circuit 42, the electronic shutter 45 sets the exposure time of the image pickup device 23B to the third exposure time suitable for the narrow band imaging and sets the exposure time of the image pickup device 23A to the fourth exposure time shorter than the third exposure time.

Images of the imaging subject 501 illuminated with a narrow band light emitted by the illumination objective system 21C are respectively focused by the objective optical systems 21A and 21B, spectrally analyzed by the spectral filters 22A and 22B, picked up by the image pickup devices 23A and 23B, and outputted as image pickup signals to the image processing circuit 43 of the controller 4.

Based on the second control signal outputted from the control circuit 42 and the image pickup signals outputted, respectively, from the image pickup devices 23A and 23B, the image processing circuit 43 processes the image pickup signals outputted from the image pickup devices 23A and 23B so that the image of the imaging subject 501 picked up by the image pickup device 23B will be displayed in larger sizes on the monitor 5 than the image of the imaging subject 501 picked up by the image pickup device 23A, and outputs the processed image pickup signals as video signals to the monitor 5.

Based on the video signals outputted from the image processing circuit 43, the monitor 5 displays the image of the imaging subject 501 picked up by the image pickup device 23A as a normal image 52A and the image of the imaging subject 501 picked up by the image pickup device 23B as a narrow band image 52B, for example, as shown in Fig. 4.

As the control, processes, and the like described above are performed by the living body image pickup apparatus 2, light source 3, and controller 4, images are displayed on the monitor 5 in a form suitable for display in narrow band imaging mode: the normal image 52A is displayed in a reduced form and the narrow band image 52B is displayed in an enlarged form at an optimum brightness. Furthermore, as the control, processes, and the like described above are performed by the living body image pickup apparatus 2, light source 3, and controller 4, the narrow band image 52B is displayed on the monitor 5 with blood vessels and microstructures in a surface layer of mucous membrane on the imaging subject in the living body being enhanced.

Incidentally, the living body imaging system 1 may be configured as a living body imaging system 1A shown in Fig. 5, the living body imaging system 1A including a living body image pickup apparatus 2A, light source 3 similar in configuration to the light source 3 described above, controller 4A, and monitor 5 similar in configuration to the monitor described above.

In addition to configuration of the living body image pickup apparatus 2, the living body image pickup apparatus 2A further comprises second narrow band imaging means, including an objective optical system 21D which forms an image of the imaging subject 501, a spectral filter 22D which has a third wavelength band and spectrally analyzes the image of the imaging subject 501 focused by the objective optical system 21D, and an image pickup device 23D such as a CCD (charge-coupled device) or the like which, serving as image pickup means, picks up the image of the imaging subject 501 spectrally analyzed by the spectral filter 22D and outputs the resulting image of the imaging subject 501 as an image pickup signal.

The spectral filter 22D serving as spectroscopic means has transmission characteristics of passing light in B2, B3, and B4 wavelength bands which, for example, ranging between 400 nm and 500 nm, have different peak wavelengths as shown in Fig. 6.

The controller 4A includes an operation panel 41A installed on an exterior surface of the controller 4A which outputs command signals in response to surgeon's actions, a control circuit 42A which performs various types of control based on the command signals outputted from the operation panel 41A, an image processing circuit 43A which processes the image pickup signals outputted from the image pickup devices 23A, 23B, and 23D under the control of the control circuit 42A, a dimmer circuit 44A which controls the quantity of light emitted by the lamp 31 of the light source 3 under the control of the control circuit 42A, and an electronic shutter 45A which adjusts exposure times of the image pickup devices 23A, 23B, and 23D.

The operation panel 41 A includes user control means such as an imaging mode changeover switch which outputs an imaging mode changeover command signal in order to switch among normal imaging mode which provides images of a desired imaging subject in a living body in a manner similar to naked-eye observations, first narrow band imaging mode which provides images with blood vessels and microstructures in a surface layer of mucous membrane on the imaging subject being enhanced, and second narrow band imaging mode which provides images with blood vessels and microstructures in the surface layer of the mucous membrane on the imaging subject in the living body being more enhanced than in the first narrow band imaging mode.

If, for example, a command for normal imaging is detected based on the command signal outputted from the operation panel 41A, the control circuit 42A serving as control means outputs a first control signal containing control details for the normal imaging mode to the image processing circuit 43A, dimmer circuit 44A, and electronic shutter 45A.

Also, if, for example, a command for the first narrow band imaging mode is detected based on the command signal outputted from the operation panel 41A, the control circuit 42A outputs a second control signal containing control details for the first narrow band imaging mode to the image processing circuit 43A, dimmer circuit 44A, and electronic shutter 45A.

Furthermore, if, for example, a command for the second narrow band imaging mode is detected based on the command signal outputted from the operation panel 41 A, the control circuit 42A outputs a third control signal containing control details for the second narrow band imaging mode to the image processing circuit 43A, dimmer circuit 44A, and electronic shutter 45A.

Based on the first control signal outputted from the control circuit 42A, the image processing circuit 43A serving as image processing means processes the image pickup signals outputted from the image pickup devices 23A, 23B, and 23D so that the image of the imaging subject 501 picked up by the image pickup device 23A will be displayed in larger sizes on the monitor 5 than the image of the imaging subject 501 picked up by the image pickup device 23B and image of the imaging subject 501 picked up by the image pickup device 23D, and outputs the processed image pickup signals to the monitor 5.

Also, based on the second control signal outputted from the control circuit 42A, the image processing circuit 43A processes the image pickup signals outputted from the image pickup devices 23A, 23B, and 23D so that the image of the imaging subject 501 picked up by the image pickup device 23B will be displayed in larger sizes on the monitor 5 than the image of the imaging subject 501 picked up by the image pickup device 23A and image of the imaging subject 501 picked up by the image pickup device 23D, and outputs the processed image pickup signals to the monitor 5.

Also, based on the third control signal outputted from the control circuit 42A, the image processing circuit 43A processes the image pickup signals outputted from the image pickup devices 23A, 23B, and 23D so that the image of the imaging subject 501 picked up by the image pickup device 23D will be displayed in larger sizes on the monitor 5 than the image of the imaging subject 501 picked up by the image pickup device 23A and image of the imaging subject 501 picked up by the image pickup device 23B, and outputs the processed image pickup signals to the monitor 5.

Based on the first control signal outputted from the control circuit 42A, the dimmer circuit 44A serving as dimming means sets the quantity of light emitted by the lamp 31 to a first emitted-light quantity so that the image of the imaging subject 501 picked up by the image pickup device 23A will be displayed on the monitor 5 at a brightness suitable for normal imaging.

Also, based on the second control signal outputted from the control circuit 42A, the dimmer circuit 44A sets the quantity of light emitted by the lamp 31 to a second emitted-light quantity larger than the first emitted-light quantity so that the image of the imaging subject 501 picked up by the image pickup device 23B will be displayed on the monitor 5 at a brightness suitable for first narrow band imaging.

Furthermore, based on the third control signal outputted from the control circuit 42A, the dimmer circuit 44A sets the quantity of light emitted by the lamp 31 to a third emitted-light quantity larger than the first emitted-light quantity, but smaller than the second emitted-light quantity so that the image of the imaging subject 501 picked up by the image pickup device 23D will be displayed on the monitor 5 at a brightness suitable for second narrow band imaging.

Based on the first control signal outputted from the control circuit 42A, the electronic shutter 45A serving as exposure adjusting means sets the exposure time of the image pickup device 23A to a first exposure time suitable for the normal imaging and sets the exposure time of the image pickup devices 23B and 23D to a second exposure time longer than the first exposure time.

Also, based on the second control signal outputted from the control circuit 42A, the electronic shutter 45A sets the exposure time of the image pickup device 23B to a third exposure time suitable for the first narrow band imaging, sets the exposure time of the image pickup device 23A to a fourth exposure time shorter than the third exposure time, and sets the exposure time of the image pickup device 23D to the third exposure time.

Furthermore, based on the third control signal outputted from the control circuit 42A, the electronic shutter 45A sets the exposure time of the image pickup device 23D to a fifth exposure time suitable for the second narrow band imaging, sets the exposure time of the image pickup device 23A to the fourth exposure time, and sets the exposure time of the image pickup device 23B to the fifth exposure time, the fifth exposure time being longer than the fourth exposure time.

Next, operation of the living body imaging system 1A according to the present embodiment will be described.

The surgeon or the like powers on various components of the living body imaging system 1A, i.e., the living body image pickup apparatus 2A, light source 3, controller 4A, and monitor 5 and thereby starts up these components. It is assumed that the living body image pickup apparatus 2A, light source 3, and controller 4A are set to enter normal imaging mode upon start-up.

When the controller 4A is set to normal imaging mode, the control circuit 42A detects based on the imaging mode changeover command signal outputted from the operation panel 41 A that normal imaging has been specified, and outputs the first control signal to the image processing circuit 43A, dimmer circuit 44A, and electronic shutter 45A. Also, when the controller 4A is set to the normal imaging mode, based on the first control signal outputted from the control circuit 42A, the dimmer circuit 44A sets the quantity of light emitted by the lamp 31 to the first emitted-light quantity so that the image of the imaging subject 501 picked up by the image pickup device 23A will be displayed on the monitor 5 at a brightness suitable for the normal imaging. Furthermore, when the controller 4A is set to the normal imaging mode, based on the first control signal outputted from the control circuit 42A, the electronic shutter 45A sets the exposure time of the image pickup device 23A to the first exposure time suitable for the normal imaging and sets the exposure times of the image pickup devices 23B and 23D to the second exposure time longer than the first exposure time.

Subsequently, the surgeon or the like moves the living body image pickup apparatus 2A to place a desired imaging subject in the living body in such a position as to come into view of the objective optical systems 2 1 A, 2 1 B, and 2 1 D and be illuminated with the illuminating light emitted from the illumination objective system 21C.

Under the conditions described above, images of the imaging subject 501 illuminated with a wide band light emitted by the illumination objective system 21C are respectively focused by the objective optical systems 2 1 A, 2 1 B, and 21D, spectrally analyzed by the spectral filters 22A, 22B, and 22D, picked up by the image pickup devices 23A, 23B, and 23D, and outputted as image pickup signals to the image processing circuit 43A of the controller 4A.

Based on the first control signal outputted from the control circuit 42A and the image pickup signals outputted, respectively, from the image pickup devices 23A, 23B, and 23D, the image processing circuit 43A processes the image pickup signals outputted from the image pickup devices 23A, 23B, and 23D so that the image of the imaging subject 501 picked up by the image pickup device 23A will be displayed in larger sizes on the monitor 5 than the images of the imaging subject 501 picked up by the image pickup devices 23B and 23D, and outputs the processed image pickup signals as video signals to the monitor 5.

Based on the video signals outputted from the image processing circuit 43A, the monitor 5 displays the image of the imaging subject 501 picked up by the image pickup device 23A as a normal image 51a, the image of the imaging subject 501 picked up by the image pickup device 23B as a first narrow band image 5 1 b, and the image of the imaging subject 501 picked up by the image pickup device 23D as a second narrow band image 51d, for example, as shown in Fig. 7.

As the control, processes, and the like described above are performed by the living body image pickup apparatus 2A, light source 3, and controller 4A, images are displayed on the monitor 5 in a form suitable for display in normal imaging mode: the normal image 51a is displayed in an enlarged form at an optimum brightness and the first narrow band image 51b and second narrow band image 51d are displayed in a reduced form. Furthermore, as the control, processes, and the like described above are performed by the living body image pickup apparatus 2A, light source 3, and controller 4A, the normal image 51 a is displayed on the monitor 5 in a manner similar to naked-eye observations of a desired imaging subject in the living body.

When the surgeon or the like gives a command, for example, to switch the imaging mode of the living body image pickup apparatus 2A, light source 3, and controller 4A from normal imaging mode to first narrow band imaging mode by manipulating the imaging mode changeover switch on the operation panel 41A, the operation panel 4A outputs an imaging mode changeover command signal to the control circuit 42A based on the command.

Based on the imaging mode changeover command signal outputted from the operation panel 41A, the control circuit 42A detects that the first narrow band imaging has been specified, and outputs the second control signal to the image processing circuit 43A, dimmer circuit 44A, and electronic shutter 45A. Also, when the controller 4A is set to the first narrow band imaging mode, based on the second control signal outputted from the control circuit 42A, the dimmer circuit 44A sets the quantity of light emitted by the lamp 31 to the second emitted-light quantity so that the image of the imaging subject 501 picked up by the image pickup device 23A will be displayed on the monitor 5 at a brightness suitable for the first narrow band imaging. Furthermore, when the controller 4A is set to the first narrow band imaging mode, based on the second control signal outputted from the control circuit 42A, the electronic shutter 45A sets the exposure times of the image pickup devices 23B and 23D to the third exposure time and sets the exposure time of the image pickup device 23A to the fourth exposure time shorter than the third exposure time.

Images of the imaging subject 501 illuminated with a narrow band light emitted by the illumination objective system 21C are respectively focused by the objective optical systems 21A, 21B, and 21D, spectrally analyzed by the spectral filters 22A, 22B, and 22D, picked up by the image pickup devices 23A, 23B, and 23D, and outputted as image pickup signals to the image processing circuit 43A of the controller 4A.

Based on the second control signal outputted from the control circuit 42A and the image pickup signals outputted, respectively, from the image pickup devices 23A, 23B, and 23D, the image processing circuit 43A processes the image pickup signals outputted from the image pickup devices 23A, 23B, and 23D so that the image of the imaging subject 501 picked up by the image pickup device 23B will be displayed in larger sizes on the monitor 5 than the images of the imaging subject 501 picked up by the image pickup devices 23A and 23D, and outputs the processed image pickup signals as video signals to the monitor 5.

Based on the video signals outputted from the image processing circuit 43A, the monitor 5 displays the image of the imaging subject 501 picked up by the image pickup device 23A as a normal image 52a, the image of the imaging subject 501 picked up by the image pickup device 23B as a first narrow band image 52b, and the image of the imaging subject 501 picked up by the image pickup device 23D as a second narrow band image 52d, for example, as shown in Fig. 7.

As the control, processes, and the like described above are performed by the living body image pickup apparatus 2A, light source 3, and controller 4A, images are displayed on the monitor 5 in a form suitable for display in first narrow band imaging mode: the narrow band image 52b is displayed in an enlarged form at an optimum brightness and the normal image 52a and second narrow band image 52d are displayed in a reduced form. Furthermore, as the control, processes, and the like described above are performed by the living body image pickup apparatus 2A, light source 3, and controller 4A, the narrow band image 52b is displayed on the monitor 5 with blood vessels and microstructures in the surface layer of the mucous membrane on the imaging subject in the living body being enhanced.

When the surgeon or the like gives a command, for example, to switch the imaging mode of the living body image pickup apparatus 2A, light source 3, and controller 4A from first narrow band imaging mode to second narrow band imaging mode by manipulating the imaging mode changeover switch on the operation panel 41 A, the operation panel 41 A outputs an imaging mode changeover command signal to the control circuit 42A based on the command.

Based on the imaging mode changeover command signal outputted from the operation panel 41 A, the control circuit 42A detects that the second narrow band imaging has been specified, and outputs the third control signal to the image processing circuit 43A, dimmer circuit 44A, and electronic shutter 45A. Also, when the controller 4A is set to the second narrow band imaging mode, based on the third control signal outputted from the control circuit 42A, the dimmer circuit 44A sets the quantity of light emitted by the lamp 31 to the third emitted-light quantity so that the image of the imaging subject 501 picked up by the image pickup device 23A will be displayed on the monitor 5 at a brightness suitable for the second narrow band imaging. Furthermore, when the controller 4A is set to the second narrow band imaging mode, based on the third control signal outputted from the control circuit 42, the electronic shutter 45A sets the exposure times of the image pickup devices 23B and 23D to the fifth exposure time and sets the exposure time of the image pickup device 23A to the fourth exposure time shorter than the third exposure time.

Images of the imaging subject 501 illuminated with a narrow band light emitted by the illumination objective system 21C are respectively focused by the objective optical systems 21A, 21B, and 21D, spectrally analyzed by the spectral filters 22A, 22B, and 22D, picked up by the image pickup devices 23A, 23B, and 23D, and outputted as image pickup signals to the image processing circuit 43A of the controller 4A.

Based on the third control signal outputted from the control circuit 42A and the image pickup signals outputted, respectively, from the image pickup devices 23A, 23B, and 23D, the image processing circuit 43A processes the image pickup signals outputted from the image pickup devices 23A, 23B, and 23D so that the image of the imaging subject 501 picked up by the image pickup device 23D will be displayed in larger sizes on the monitor 5 than the images of the imaging subject 501 picked up by the image pickup devices 23A and 23B, and outputs the processed image pickup signals as video signals to the monitor 5.

Based on the video signals outputted from the image processing circuit 43A, the monitor 5 displays the image of the imaging subject 501 picked up by the image pickup device 23A as a normal image 53a, the image of the imaging subject 501 picked up by the image pickup device 23B as a first narrow band image 53b, and the image of the imaging subject 501 picked up by the image pickup device 23D as a second narrow band image 53d, for example, as shown in Fig. 7.

As the control, processes, and the like described above are performed by the living body image pickup apparatus 2A, light source 3, and controller 4A, images are displayed on the monitor 5 in a form suitable for display in second narrow band imaging mode: the narrow band image 53d is displayed in an enlarged form at an optimum brightness and the normal image 53a and first narrow band image 53b are displayed in a reduced form. Furthermore, as the control, processes, and the like described above are performed by the living body image pickup apparatus 2A, light source 3, and controller 4A, the second narrow band image 53d is displayed on the monitor 5 with blood vessels and microstructures in the surface layer of the mucous membrane on the imaging subject in the living body being more enhanced than in the first narrow band image 52b.

As described above, both the living body imaging system 1 and living body imaging system 1 A include the living body image pickup apparatus which contains spectral filters. This allows the surgeon and the like to perform narrow band imaging using the living body imaging system 1 equipped with the living body image pickup apparatus 2 or living body imaging system 1A equipped with the living body image pickup apparatus 2A without using a dedicated light source. This makes it possible to reduce costs for narrow band imaging more than before.

It should be noted that the present invention is not limited to the embodiment described above, and it goes without saying that various modifications and applications are possible without departing from the spirit of the present invention.

This application is based upon and claims the benefit of priority from the prior Japanese Patent Application No. 2005-307620, filed on October 21, 2005, the entire contents of which are incorporated in the specification, claims, and drawings herein by reference.

## Claims

1. A living body image pickup apparatus comprising:
a plurality of image pickup means which pick up images of body tissue illuminated by a white light and output the images of the body tissue as image pickup signals;
first spectroscopic means which, having transmission characteristics of passing light in a first wavelength band, spectrally analyzes an image of the body tissue picked up by first image pickup means out of the plurality of image pickup means and thereby allows the image of the body tissue to be displayed on display means as a first image; and
second spectroscopic means which, having transmission characteristics of passing light in a second wavelength band different from the first wavelength band, spectrally analyzes an image of the body tissue picked up by second image pickup means out of the plurality of image pickup means and thereby allows the image of the body tissue to be displayed on the display means as a second image in which predetermined part of the body tissue is enhanced compared to the first image.

2. The living body image pickup apparatus according to claim 1, further comprising third spectroscopic means which, having transmission characteristics of passing light in a third wavelength band different from the first wavelength band and the second wavelength band, spectrally analyzes an image of the body tissue picked up by third image pickup means out of the plurality of image pickup means and thereby allows the image of the body tissue to be displayed on the display means as a third image in which predetermined part is enhanced compared to the second image.

3. The living body image pickup apparatus according to claim 1 or 2, wherein the predetermined part is blood vessels.

4. The living body image pickup apparatus according to any one of claims 1 to 3,
wherein the light in the first wavelength band includes a red band, a first green band, and a first blue band.

5. The living body image pickup apparatus according to claim 4, wherein the light in the second wavelength band includes a second green band narrower than the first green band and a second blue band narrower than the first blue band.

6. The living body image pickup apparatus according to claim 4 or 5, wherein the light in the third wavelength band has a plurality of peak wavelengths in the first blue band.

7. A living body imaging system comprising: a light source which emits a white light as an illuminating light to illuminate an imaging subject in a living body; a living body image pickup apparatus which pick up images of the imaging subject and output the images of the imaging subject as an image pickup signal; and a controller which controls the light source and the living body image pickup apparatus,
wherein the living body image pickup apparatus comprises a plurality of image pickup means which pick up images of the imaging subject and output the images of the imaging subject as image pickup signals, first spectroscopic means which, having transmission characteristics of passing light in a first wavelength band, spectrally analyzes an image of the imaging subject picked up by first image pickup means out of the plurality of image pickup means and thereby allows the image of the imaging subject to be displayed on display means as a first image, and second spectroscopic means which, having transmission characteristics of passing light in a second wavelength band different from the first wavelength band, spectrally analyzes an image of the imaging subject picked up by second image pickup means out of the plurality of image pickup means and thereby allows the image of the imaging subject to be displayed on the display means as a second image in which predetermined body tissue in the imaging subject is enhanced compared to the first image, and
the controller comprises user control means which outputs an imaging mode switching signal to switch between a first imaging mode for imaging of the first image and a second imaging mode for imaging of the second image, and control means which outputs a control signal if it is detected based on the imaging mode switching signal that imaging in one of the imaging modes is specified from the user control means, the control signal being intended to adjust exposure time of each of the plurality of image pickup means, control light quantity of the white light emitted by the light source means, and change display state of the first image and the second image on the display means, according to the specified imaging mode.

8. The living body imaging system according to claim 7, wherein the predetermined part is blood vessels.

9. The living body imaging system according to claim 7 or 8, wherein the light in the first wavelength band includes a red band, a first green band, and a first blue band.

10. The living body imaging system according to claim 9, wherein the light in the second wavelength band includes a second green band narrower than the first green band and a second blue band narrower than the first blue band.

## Amended claims

### Amended claims under Art. 19.1 PCT

**1.** A living body image pickup apparatus comprising:
a plurality of image pickup means which pick up images of body tissue illuminated by a white light and output the images of the body tissue as image pickup signals;
first spectroscopic means which, having transmission characteristics of passing light in a first wavelength band, spectrally analyzes an image of the body tissue picked up by first image pickup means out of the plurality of image pickup means and thereby allows the image of the body tissue to be displayed on display means as a first image; and
second spectroscopic means which, having transmission characteristics of passing light in a second wavelength band different from the first wavelength band, spectrally analyzes an image of the body tissue picked up by second image pickup means out of the plurality of image pickup means and thereby allows the image of the body tissue to be displayed on the display means as a second image in which predetermined part of the body tissue is enhanced compared to the first image.

**2.** The living body image pickup apparatus according to claim 1, further comprising third spectroscopic means which, having transmission characteristics of passing light in a third wavelength band different from the first wavelength band and the second wavelength band, spectrally analyzes an image of the body tissue picked up by third image pickup means out of the plurality of image pickup means and thereby allows the image of the body tissue to be displayed on the display means as a third image in which predetermined part is enhanced compared to the second image.

**3.** The living body image pickup apparatus according to claim 1 or 2, wherein the predetermined part is blood vessels.

**4.** The living body image pickup apparatus according to any one of claims 1 to 3,
wherein the light in the first wavelength band includes a red band, a first green band, and a first blue band.

**5.** The living body image pickup apparatus according to claim 4, wherein the light in the second wavelength band includes a second green band narrower than the first green band and a second blue band narrower than the first blue band.

**6.** (Amended) The living body image pickup apparatus according to claim 2,
wherein the light in the third wavelength band has a plurality of peak wavelengths in the first blue band.

**7.** A living body imaging system comprising: a light source which emits a white light as an illuminating light to illuminate an imaging subject in a living body; a living body image pickup apparatus which pick up images of the imaging subject and output the images of the imaging subject as an image pickup signal; and a controller which controls the light source and the living body image pickup apparatus,
wherein the living body image pickup apparatus comprises a plurality of image pickup means which pick up images of the imaging subject and output the images of the imaging subject as image pickup signals, first spectroscopic means which, having transmission characteristics of passing light in a first wavelength band, spectrally analyzes an image of the imaging subject picked up by first image pickup means out of the plurality of image pickup means and thereby allows the image of the imaging subject to be displayed on display means as a first image, and second spectroscopic means which, having transmission characteristics of passing light in a second wavelength band different from the first wavelength band, spectrally analyzes an image of the imaging subject picked up by second image pickup means out of the plurality of image pickup means and thereby allows the image of the imaging subject to be displayed on the display means as a second image in which predetermined body tissue in the imaging subject is enhanced compared to the first image, and
the controller comprises user control means which outputs an imaging mode switching signal to switch between a first imaging mode for imaging of the first image and a second imaging mode for imaging of the second image, and control means which outputs a control signal if it is detected based on the imaging mode switching signal that imaging in one of the imaging modes is specified from the user control means, the control signal being intended to adjust exposure time of each of the plurality of image pickup means, control light quantity of the white light emitted by the light source means, and change display state of the first image and the second image on the display means, according to the specified imaging mode.

**8.** The living body imaging system according to claim 7, wherein the predetermined part is blood vessels.

**9.** The living body imaging system according to claim 7 or 8, wherein the light in the first wavelength band includes a red band, a first green band, and a first blue band.

**10.** The living body imaging system according to claim 9, wherein the light in the second wavelength band includes a second green band narrower than the first green band and a second blue band narrower than the first blue band.
